# EUROPÄISCHE PATENTSCHRIFT

(11) **EP 0 872 234 B1**
(45) Veröffentlichungstag und Bekanntmachung des Hinweises auf die Patenterteilung: **16.03.2005**
(21) Anmeldenummer: 98106347.2
(22) Anmeldetag: 07.04.1998
(51) Int. Cl.: A61K 9/70

(54) **Laminare Arzneiform**
Laminar medical formulation
Forme médicale laminaire

(30) Priorität: 16.04.1997 DE 19715794
(43) Veröffentlichungstag der Anmeldung: 21.10.1998
(73) Patentinhaber: Röhm GmbH & Co. KG, 64293 Darmstadt (DE)
(72) Erfinder: Lehmann, Klaus, Dr., 64380 Rossdorf (DE); Petereit, Hans-Ulrich, 64291 Darmstadt (DE)

(56) Entgegenhaltungen:
- DE-A- 2 656 387
- US-A- 3 625 214

## Beschreibung

Die Erfindung betrifft laminare Arzneiformen aus gerollten oder gefalteten wirkstoffhaltigen Polymerfolien.

### Stand der Technik

Das Einrollen von wirkstoffhaltigen Polymerfilmen bzw. -folien zu Arzneiformen ist bekannt. EP 0 086 997 und EP 0 122 574 beschreiben die Einbettung von Wirkstoffen in Polymerfilmen zur retardierten Freisetzung, doch werden diese Filme einzeln oder in zerkleinerter Form in Kapseln oder durch Verpressen zu Tabletten verarbeitet. Die Retardierung wird im wesentlichen durch die Art des Films und die Größe seiner Oberfläche bestimmt. Beim Beginn der Wirkstoffabgabe steht hier alsbald die gesamte Filmoberfläche im Kontakt mit der Verdauungsflüssigkeit und damit zur Wirkstofffreisetzung zur Verfügung.

EP-A 010 987 beschreibt das Aufrollen eines wirkstoffhaltigen Films zur Applikation von veterinärmedizinischen Medikamenten bei Wiederkäuern, wobei sich die primäre Form nach Applikation entrollen und zu einer vergrößerten sekundären Form aufweiten soll. Der Zweck der Volumenvergrößerung ist der, daß die Arzneiform dadurch längere Zeit im Rumen verbleibt. Die Wirkstoffabgabe erfolgt dann auch hier aus der allseitig den Verdauungssäften zugänglichen, gesamten Filmoberfläche. In ähnlicher Weise soll ein Laminat wirken, das nach EP-A 363 187 mit einem druckempfindlichen Kleber hergestellt wird. Dies ist auch beschrieben in EP-A 350 188.

Es ist auch vorgeschlagen worden, aufgerollte, wirkstoffhaltige Filme in Körperhöhlen einzubringen EP-A 447 719 und zwar zur Therapie von Mittelohrinfektionen oder nach JP-Abstract 82-90967 (Derwent) intravaginal zur Verabreichung spermizider Mittel als Kontrazeptivum. In diesen Fällen ist nur eine örtliche Wirkstoffabgabe beabsichtigt, wobei man auch hier davon ausgehen kann, daß die Filmrolle alsbald durch Feuchtigkeitsaufnahme expandiert und der Wirkstoff aus dem gelockerten Filmverband abgegeben wird.

Eine im Magen aufschwimmende Arzneiform ist in EP-A 326 816 beschrieben. Hierbei werden mit Wirkstoff angereicherte Polymerfilme zu verschiedenen Formen verarbeitet, die jedoch durch Mitverwendung poröser Materialien oder einen Einschluß von Luft eine längere Verweilszeit im Magen haben sollen. Offenbar in Fortentwicklung dieser Idee sind in DE 4419818 auch gerollte Filme erwähnt, die durch Entfaltung im Magen längere Zeit verbleiben und der Pyloruspassage wegen ihrer Größe von mehr als 5 cm² nur schwer zugänglich sein sollen. Hier wird ebenfalls nach Entfaltung bzw. Aufweitung auch die gesamte Oberfläche des Films für die Wirkstoffabgabe zugänglich.

US 3,625,214 beschreibt eine Zusammensetzung zur verzögerten Wirkstoffabgabe, die aus einem spiralförmig gerollten Substrat besteht. Dieses setzt sich aus einem flexiblen, in Körperflüssigkeiten erodierbaren Film zusammen, der mit einer erodierbaren wirkstoffhaltigen Matrix beschichtet ist.

DE 26 56 387 A1 beschreibt eine feste pharmazeutische Einheitsdosierungsform als Darreichungsform eines Medikaments sowie Verfahren und Anlage zu ihrer Herstellung.

### Aufgabe und Lösung

Als Aufgabe der vorliegenden Erfindung wird die Bereitstellung einer Alternative zu herkömmlichen Arzneiformen, bestehend aus wirkstoffhaltigem Kern und Filmhülle oder schwammartiger Matrixstruktur, gesehen.

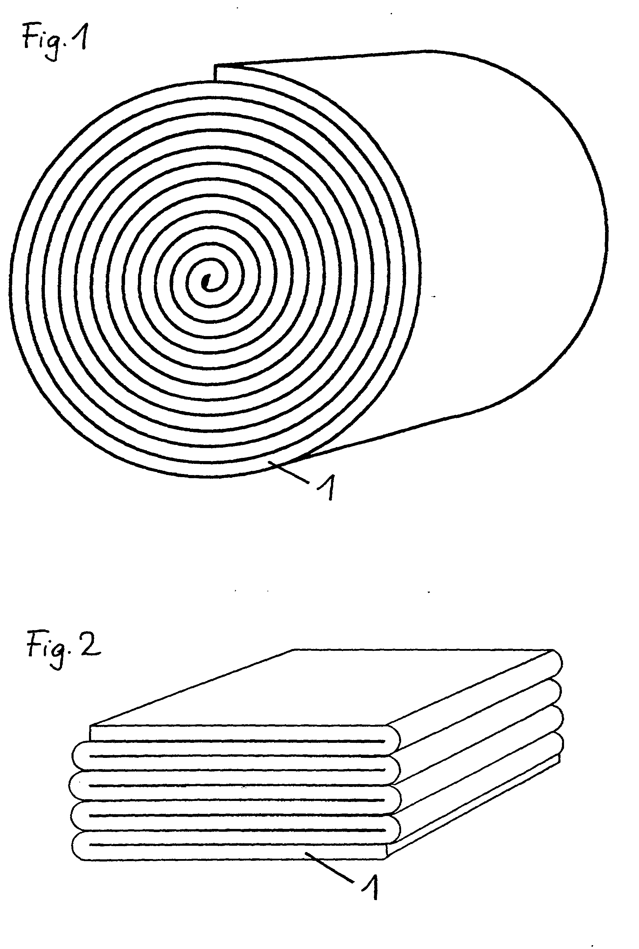

Die Aufgabe wurde gelöst durch eine laminare Arzneiform mit kontrollierter Wirkstoffabgabe, bestehend aus gerollten oder gefalteten Schichten einer Polymerfolie (1, siehe Figur 1 - 3), die einen pharmazeutischen Wirkstoff enthält,
dadurch gekennzeichnet, dass
die wirkstoffhaltige Polymerfolie (1) aus einem (Meth)acrylat-Copolymer, aus Celluloseacetatphthalat, Hydroxypropylmethylcellulosephthalat, Carboxymethylcellulose, Polyvinylacetatphthalat, gegebenenfalls mit Zusätzen von Weichmachern oder Bindemitteln besteht und
die äußere, den Verdauungssäften zugängliche Oberfläche der wirkstoffhaltigen Polymerfolie im gerollten oder gefalteten Zustand höchstens 25 % ihrer gesamten Oberfläche beträgt und die gerollten oder gefalteten Schichten so aneinander haften, daß die laminare Arzneiform im Freigabetest nach USP 23, Methode A, Apparatus 2 bei 37 °C und 50 Upm in künstlichem Magensaft ihre gerollte oder gefaltete Form über mindestens eine Stunde beibehält und mindestens 30 % des enthaltenen Wirkstoffs im gerollten oder gefalteten Zustand freigesetzt werden.

Das Prinzip der Erfindung liegt darin, daß durch den laminaren Aufbau der Arzneiform eine sehr gleichmäßige Struktur entsteht, wobei nur eine geringe Oberfläche der wirkstoffhaltigen Polymerfolie Kontakt zu den Verdauungsflüssigkeiten hat. Der laminare Aufbau bedingt, daß die Wirkstoffdiffusion in die Verdauungsflüssigkeit im wesentlichen nur an den Schnittkanten und in den Schichten und aus ihnen heraus über einen längeren Diffusionsweg erfolgt, während zwischen den Schichten keine oder nur eine geringe Diffusion stattfindet. Die Wirkstoffdiffusion folgt daher dem laminaren Aufbau, wodurch es möglich ist, in vielfältiger Weise, eine verzögerte, kontrollierte Wirkstofffreisetzung einzustellen.

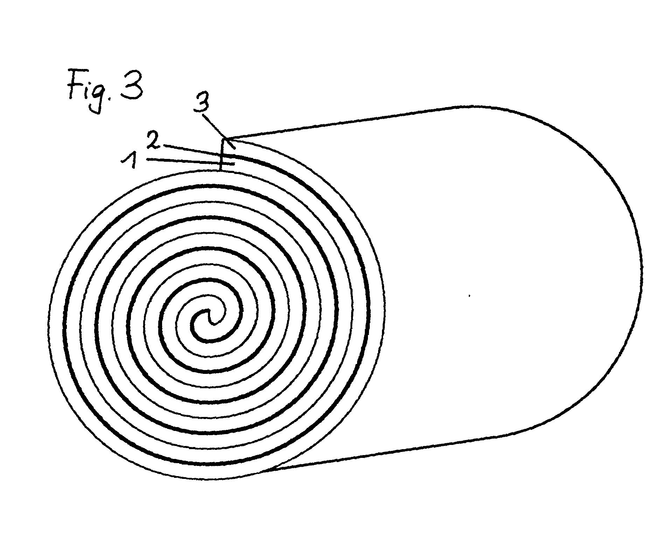

In einer bevorzugten Ausführungsform wird die wirkstoffhaltige Polymerfolie vor dem Einrollen oder Falten noch mit einer oder zwei weiteren Schichten versehen. Die weitere Schicht kann dabei als Zwischenschicht ((3) s. Fig 3) die Funktion ausüben, die Diffusion zwischen den wirkstoffhaltigen Schichten zu reduzieren oder zu verhindern und so zur Steuerung einer kontrollierten Wirkstoffabgabe beitragen. Auch kann eine weitere Schicht zusätzlich eine Klebefunktion ((2) s. Fig. 3) ausüben, die zum Zusammenhalt der laminaren Arzneiform in Gegenwart der Verdauungsflüssigkeiten über einen längeren Zeitraum beiträgt, wenn die Polymerfilme unter den Bedingungen des Zusammenrollens oder -faltens nicht genügend aneinander haften.

Eine weitere erfinderische Ausgestaltung besteht darin, die laminare Arzneiform mit einer Banderole zu versehen, die einerseits zum Zusammenhalt der laminaren Arzneiform beiträgt und andererseits die Diffusion von Wirkstoff aus der zu äußerst liegenden Polymerfolienschicht weitgehend unterbindet.

Gegenüber konventionellen Arzneiformen mit wirkstoffhaltigen Kern und einer die Wirkstofffreisetzung steuernden Filmhülle ergibt sich der Vorteil des einfacheren sehr gleichmäßigen Aufbaus und der einfacheren Herstellbarkeit, nach den in den letzten Jahren sehr weit entwickelten rationellen Verfahren der Film- bzw. Folienherstellung durch Beschichten, Extrudieren, "Hot-Melt"-Verfahren, wie sie bei der Herstellung von transdermalen Arzneiformen in steigendem Maße angewendet werden. Auf einen Kern kann völlig verzichtet werden. Ebenso sind aufwendige und kritische Coatingverfahren nicht vonnöten.

Die Erfindung wird durch die nachstehenden Figuren erläutert, ist aber nicht auf die dargestellten Ausführungsformen beschränkt.
Fig. 1:
   Schematische Darstellung einer laminare Arzneiform aus einer gerollten wirkstoffhaltigen Polymerfolie (1).
Fig. 2:
   Schematische Darstellung einer laminare Arzneiform aus einer gefalteten wirkstoffhaltigen Polymerfolie (1).
Fig. 3:
   Schematische Darstellung einer gerollten laminaren Arzneiform mit einer wirkstoffhaltigen Polymerfolie (1), einer Klebeschicht (2) und einer Zwischenschicht (3).

### Ausführung der Erfindung

Unter einer Polymerfolie im Sinne der Erfindung ist ein roll- oder faltbare Folie von geringer Dicke aus einem (Meth)acrylat-Copolymer, aus Celluloseacetatphthalat, Hydroxypropylmethylcellulosephthalat, Carboxymethylcellulose, Polyvinylacetatphthalat, gegebenenfalls mit Zusätzen von Weichmachern oder Bindemitteln zu verstehen. Die Folie kann auch aus einer Mischung verschiedener Polymere bestehen.

Die laminare Arzneiform soll ihre gerollte oder gefaltete Form in Gegenwart von Verdauungsflüssigkeit, Magen und/oder Darmsäften, über einem längeren Zeitraum beibehalten. Unter einem längeren Zeitraum ist zu verstehen, daß ein Entrollen oder Entfalten erst dann erfolgen soll, wenn die Hauptmenge des enthaltene Wirkstoffs bereits im wesentlichen über die laminaren Schichten der äußeren Oberfläche freigesetzt wurde.

Diese Bedingung ist abhängig von der beabsichtigten therapeutischen Anwendung. Als grober Richtwert für den genannten längeren Zeitraum kann gelten, daß die Magenpassagezeit bei üblicher Nahrungsaufnahme von ca. 1 - 2 Stunden nicht unterschritten werden soll und daß während des längeren Zeitraums in der die gerollte oder gefaltete Form beibehalten wird, mehr als 30 % des enthaltenen Wirkstoffs freigesetzt werden soll. Bevorzugt liegt der längere Zeitraum jedoch im Bereich von 3 - 6 Stunden oder darüber und die im wesentlichen "laminar" freigesetzte Menge des Wirkstoffs beträgt mindestens 50 %, insbesondere mindestens 60 %.

Diese in vivo-Bedingungen können als erfüllt gelten, wenn die laminare Arzneiform im in-vitro Freigabetest nach USP 23 (1994, S. 1795 ff), Methode A, Apparatus 2 ("Paddle", s. S. 1792) bei 37 °C und 50 Upm, in künstlichem Magensaft (0,1 M HCl), ihre gerollte oder gefaltete Form über mindestens eine Stunde, bevorzugt mindestens 2 Stunden, beibehält und mindestens 30 %, bevorzugt mindestens 50 %, besonders bevorzugt mindestens 60 %, des enthaltenen Wirkstoffs im gerollten oder gefalteten Zustand freigesetzt werden.

Auf keinen Fall soll sich die laminare Arzneiform bereits unmittelbar nach der Einnahme entrollen oder entfalten, da dann das Prinzip der verzögerten und kontrollierten Wirkstoffabgabe nicht ausreichend gegeben wäre (und dann auch mit einfachen Arzneiformen des Standes der Technik realisierbar wäre). Je nach therapeutischer Anwendung kann es gewünscht sein, daß zunächst die Freisetzung des Wirkstoffs über einen längeren Zeitraum im wesentlichen "laminar" erfolgt, daß dann aber die Restmenge des Wirkstoffs freigesetzt wird, indem sich sich die laminare Arzneiform entrollt oder auflöst.

Geeignete Polymere zum Aufbau der laminaren Arzneiform können ganz allgemein danach ausgewählt werden, daß sie in Folienform verfügbar sind oder hergestellt werden können und eine ausreichende Flexibilität haben, um beim Rollen oder Falten nicht zu brechen oder einzureißen. Natürlich muß das Polymermaterial für pharmazeutische Anwendungen geeignet sein und ein ausreichendes Aufnahmevermögen für den Wirkstoff besitzen. Als fertig verfügbare Folien sind z. B. Folien aus Papier oder Polyvinylacetat zu nennen. Eine Vielzahl weiterer Polymermaterialien sind als Feststoffe, z. B. als Granulat, oder im Form von Lösungen oder Dispersionen erhältlich, die sich durch Beschichten von Trägerfolien, Extrudieren oder "Hot-Melt"-Verfahren in Filme überführen lassen (siehe dazu z. B. EP-A 704 207, EP-A 704 208, EP-A 727 205 oder EP-A 704 205).
Vorzugsweise werden Polymermaterialien verwendet, die sich unter den Bedingungen des Verdauungstraktes zuletzt auflösen oder zerfallen. Hierzu kann man Polymere verwenden, die sich zum Überziehen von Arzneiformen bewährt haben. Dazu gehören magensaftlösliche Poly(meth)acrylate, Z. B. Copolymere aus 25 Gew.-% Methylmethacrylat, 25 Gew.-% Butylmethacrylat und 50 Gew.-% Dimethylaminoethylmethacrylat (Typ ®EUDRAGIT E 100), die unterhalb pH 5 im Verdauungstrakt löslich und oberhalb pH 5 quellbar und permeabel sind. Weiterhin sind magensaftresistente, aber darmsaftlösliche Copolymere der Methacrylsäure, z. B. Copolymere aus 50 Gew.-% Ethylacrylat oder Methylmethacrylat und 50 Gew.-% Methacrylsäure (® EUDRAGIT US-Typen) zu nennen. Ebenso kommen auch quellbare Polymethacrylate auf Basis von Copolymeren, die 2-Trimethylammoniumethylmethacrylat in Frage. Hier sind Polymere aus z. B. 30 Gew.-% Ethylacrylat 5 oder 10 Gew.-% 2-Trimethylammoniumethylmethacrylat und einem Restanteil von Methylmethacrylat zu nennen (EUDRAGIT® RS, EUDRAGIT® RL). Weiterhin geeignet sind Copolymere aus Methylacrylat, Methylmethacrylat und Methacrylsäure (siehe z. B. EP-A 704 207, EP-A 704 208).

Daneben sind auch Cellulosederivate mit ähnlichen Eigenschaften brauchbar wie Celluloseacetatphthalat, Hydroxypropylmethylcellulosephthalat, Carboxymethylcellulose und ähnliche Verbindungen, die oberhalb pH 5 im Darmtrakt löslich sind, ebenso Polyvinylacetatphthalat.

Zur Bereitstellung der erfindungsgemäßen Arzneiform ist zunächst die Präparation einer wirkstoffhaltigen Polymerfolie erforderlich. Dies kann in an sich bekannter Weise vorgenommen werden. Die Polymere können z. B. in organischer Lösung oder in wäßriger Dispersion vorliegen. Der Wirkstoff kann dann direkt, ggf. mit weiteren Hilfsstoffen, wie z. B. Weichmachern, zugegeben werden. Die wirkstoffhaltige Suspension bzw. Lösung kann in einer dünnen Schicht verteilt werden und bildet nach dem Verdampfen des Lösungmittels ein dünnen Film von z. B. 0,03 bis 1 mm Dicke, bevorzugt im Bereich von 0,1 bis 0,5 mm. Dabei sollen die Schichtdicken als Richtwert bei dünnen Polymerfilmen im Bereich unter ca. 0,15 mm möglichst um nicht mehr als +/- 25 % und bei dickeren Filmen um nicht mehr als um ca. +/- 10 % schwanken.

Alternativ kann der wirkstoffhaltige Polymerfilm auch lösungsmittelfrei aus einer Polymerschmelze in sogenannten "Hot-Melt"-Verfahren erhalten werden, indem das Polymer, das ggf. noch Fließverbesserer, z. B. Wachse, Cetylalkohol etc. enthält, bei einer Temperatur oberhalb seiner Glastemperatur verflüssigt wird und als dünner Film ausgebreitet oder extrudiert wird (siehe dazu z. B. EP-A 727 205, EP-A 704 205).

Der wirkstoffhaltige Film kann im einfachsten Fall direkt zu einer laminaren Arzneiform gerollt oder gefaltet werden. Dies setzt voraus, daß der wirkstoffhaltige Polymerfilm eine ausreichende Haftung zu sich selbst aufweist, so daß es in Gegenwart der Verdauungsflüssigkeiten nicht zu einem vorzeitigen Entfalten oder Entrollen kommt. Ein geeignetes Polymer ist hierfür ist z. B. ein Copolymerisat aus Ethylacrylat und Methylmethacrylat im Verhältnis 2 : 1 (EUDRAGIT® NE).

Bei dieser einfachsten Form der laminaren Arzneiform ist zu berücksichtigen, daß die Diffusion des Wirkstoffs zwar im wesentlichen laminar, also entlang der inneren Schichten erfolat, daß aber auch eine direkte Diffusion über die Oberfläche der äußerster Schicht der letzten Windung erfolgt und auch immer ein gewisser Anteil des Wirkstoffs im Innern von Schicht zu Schicht diffundiert. Dieser manchmal unerwünschte Effekt ist natürlich abhängig von der Anzahl der Schichten und deren Packungsdichte, dem verwendeten Polymer und dem eingesetzen Wirkstoff. In vielen Fällen wird dieser Effekt jedoch vernachlässigbar gering oder unerheblich sein.

Will man die Diffusion über die äußerste an der Oberfläche liegende Schicht oder die Diffusion zwischen den wirkstoffhaltigen Schichten reduzieren, so können auf die wirkstoffhaltige Polymerfolie noch ein- oder zweiseitig eine bzw. zwei weitere Schichten aufgebracht werden. Die können die Funktion einer Zwischen- oder Trennschicht für die wirkstoffhaltigen Schichten ausüben oder auch die Funktion einer Klebeschicht haben, die den Zusammenhalt der laminaren Arzneiform über längere Zeit in Gegenwart der Verdauungsflüssigkeit bewirkt oder unterstützt.

Bei der Herstellung der laminaren Arzneiform wird der wirkstoffhaltige Polymerfilm in der Regel zunächst über seine gesamte Herstellungsbreite gefaltet oder gerollt. Dies soll so geschehen, daß die Schichten einen innigen Kontakt zueinander erhalten, also z. B. unter leichten Druck. Danach wird die erhaltene Rolle oder der Faltenstapel, die einen Durchmesser bzw. eine Höhe von z. B. ca. 0,5 bis 5 cm, günstigerweise von ca. 2 - 4 cm, ausweisen kann, in viele scheibenartige Einzelportionen geschnitten. Die Breite der fertigen laminaren Arzneiformen kann z. B. im Bereich von 0,5 - 2 cm liegen und bestimmt dann durch das Flächenverhältnis der Schnittkanten zur inneren Filmoberfläche in erheblichem Maße die Wirkstoffabgabegeschwindigkeit.

Um die gewünschte Freigabe-Geschwindigkeit zu ermöglichen, ist es erforderlich, daß die äußere, den Verdauungssäften zugängliche Oberfläche der wirkstoffhaltigen Polymerfolie im gerollten oder gefalteten Zustand höchstens 25 %, vorzugsweise höchstens 5 %, der Oberfläche der Polymerfolie beträgt. Die Reduktion der Oberfläche ergibt sich durch das Verhältnis von Schichtdicke der Polymerfolie zur Zahl ihrer Wicklungen bzw. Faltungen und deren Größe (Durchmesser der Rolle bzw. Höhe des gefalteten Stapels). Die gerollten oder gefalteten Schichten müssen so aneinander haften, daß die laminare Arzneiform in Gegenwart von Verdauungsflüssigkeiten ihre gerollte oder gefaltete Form über einen längeren Zeitraum beibehält, so daß Wirkstoff im wesentlichen nur "laminar" über die äußere Oberfläche der Schnittkanten freigesetzt wird.

Ist die Haftung des Laminats in sich nicht ausreichend oder soll es ohne eine Klebeschicht einfach zusätzlich stabilisiert werden, kann die Rolle bzw. der Faltenstapel auch durch eine außen umlaufende und fest sitzende Banderole gesichert werden. Als Material hierfür kann z. B. ein Methacrylat-Copolymer oder ein mit einer Klebeschicht versehener Filmstreifen verwendet werden. Als Klebeschicht kann ein Dimethylpolysiloxan, ein Acrylatklebstoff oder ein Klebstoff auf Basis von Naturstoffen verwendet werden.
In einer weiteren erfinderischen Ausgestaltung kann der wirkstoffhaltige Polymerfilm noch vor dem Falten oder Rollen mit einer oder auch mehreren zusätzlichen Schichten versehen werden. Diese können die Funktion einer Klebeschicht und/oder einer Schicht zur Regulierung der Wrkstoffabgabe erfüllen. Auch kann die weitere Schicht ggf. dazu verwendet werden, um einen späteren Zerfall der laminaren Arzneiform zu steuern.
Eine weitere Schicht kann als Zwischenschicht den Austritt von Wirkstoff aus der wirkstoffhaltigen Schicht verzögern, bzw. regulieren. Ist sie z. B. stark hydrophob, so ist das Eindringen und Durchdringen von gelöstem hydrophilen Wirkstoff erschwert. Ist sie hydrophil, in Wasser quellbar oder sogar wasserlöslich, so wird die Wirkstoffabgabe beschleunigt bis hin zu einem Zerfall bzw. einer Auflösung der Arzneiform insgesamt. Geeignete Zwischenschichten können auch aus vorgefertigten Folien, aus z. B. Celluloseacetat, Polyvinylacetat, Polyvinylalkohol oder Polyethylen bestehen, auf die dann die wirkstoffhaltige Polymerschicht aufgebracht wird.

Eine weitere Schicht kann z. B. eine Klebeschicht sein, die den Zusammenhalt der laminaren Arzneiform bewirkt oder zumindest unterstützt. z. B. wenn die Haftung des wirkstoffhaltigen Polymerfilms zu sich selbst oder zu einer weiteren Zwischenschicht nicht ausreichend ist. Geeignete Klebeschichten können z. B. aus Dimethylpolysiloxan oder z. B. aus Butylacrylat- und Ethylhexylacrylat-Copolymeren bestehen. Weitere geeignete Klebeschichten leiten sich von Zusammensetzungen für Hauthaftkleber oder transdermale Pflaster ab (siehe z. B. EP-A 315 218, EP-A 617 972). EP-A 415 055 beschreibt beispielsweise als Klebeschicht geeignete Hauthaftkleber aus kationischen (Meth)acrylatcopolymeren, die mit einer organische Carbonsäure, z. B. Adipinsäure oder Laurinsäure, formuliert sind.

Eine gerollte laminare Arzneiform kann aus der wirkstoffhaltigen Polymerfolie, die noch eine Zwischenschicht und/oder eine Klebeschicht aufweisen kann z. B. in der Apotheke von Hand hergestellt werden. Dazu kann die Folie, die z. B. eine 50 x 50 cm bei einer Dicke von z. B. 0,2 mm messen kann, auf einer ebenen Oberfläche mit Hilfe von oben darüber geschobenen ebenen Platte unter leichtem Druck eingerollt werden, so daß eine 50 cm breite Rolle mit einem Durchmesser von z.B. 0,2 - 1 cm. Nun können z. B. 0,5 - 1 cm breite Stücke abgeschnitten werden.

Im industriellen Maßstab werden Aufrollverfahren in vielfältiger Weise bei der Herstellung und Konfektionierung von Folien benutzt. Auch bei der Fertigung von kleinen Kondensatoren werden Folien mit hoher Präzision in kleine Röllchen überführt. Solche bekannten Aufrollverfahren bzw. dazu geeignete Vorrichtungen können an die herzustellenden laminaren Arzneiformen angepaßt bzw. konstruiert werden, so daß der Einrollvorgang und das Schneiden der Rolle maschinell vorgenommen werden kann. In ähnlicher Weise können auch gefaltete laminare Arzneiformen manuell oder maschinell hergestellt werden.

Gegebenenfalls kann der Zusammenhalt der laminaren Arzneiform dadurch verbessert werden, daß das Schneiden in Portionen mit Hilfe eines heißen Drahtes oder Messers oder auch mit Hilfe eines Laserstrahls erfolgt, so daß das Polymer an den Schnittkanten leicht versintert wird. Falls eine Banderole vorgesehen ist kann diese ebenfalls manuell oder maschinell ausgebracht werden.

Die Erfindung betrifft demnach ein Verfahren zur Herstellung einer laminaren Arzneiform, wobei die wirkstoffhaltige Polymerfolie und ggf. die Zwischen- und/oder Klebeschichten gerollt oder gefaltet werden und die Schnittflächen und Enden jeweils durch heißes Schneiden versintert werden.

### BEISPIELE

Die folgenden Beispiele dienen der Erläuterung der Erfindung.

### Beispiel 1:

3 g (für Gramm bzw. Milligramm g oder mg) Theophyllin-Pulver werden in 30 g einer wäßrigen Kunststoffdispersion eingerührt. Die Kunststoffdispersion enthält 9 g eines Copolymerisates aus 65 Gewichts-% Methylacrylat, 25 Gewichts-% Methylmethacrylat und 10 Gewichts-% Methacrylsäure. Zu der Mischung werden noch 0,45 g Triethylcitrat als Weichmacher und 12 g Wasser zugesetzt. Es wird noch 5 Minuten gerührt und die Mischung dann in einen mit Polyethylenfolie ausgekleideten Trog von 16 x 16 cm Oberfläche gegeben und bei 50° C bis zur vollständigen Verfilmung getrocknet, so daß ein Film von etwa 0,5 mm Dicke entsteht.

Aus dem Film werden Streifen von 2,5 cm Breite und 10 cm Länge geschnitten und darauf ein beidseitig mit Kleber beschichtete Schaumstoffolie von 1 mm Dicke gepreßt. Die verbundenen Filme werden nun unter leichtem Druck zu einem 2,5 cm breiten Röllchen von etwa 12 mm Durchmesser gerollt und zur Fixierung zuletzt noch mit einem einseitig beschichteten Tesafilm als Banderole umklebt.

Aus dem Röllchen schneidet man mit einem scharfen Messer Scheibchen von 6 mm Dicke ab, die in dieser Form als Retardarzneimittel eingenommen werden können. Ein Scheibchen enthält 72 mg Theophyllin und gibt den Wirkstoff in einem in vitro-Freigabetest nach USP (Paddle-Gerät) bei 100 Umdrehungen/Minute und 37° C in Phosphatpuffer, pH 7,5 nach 3 Stunden 10 %, nach 6 Stunden 58 % und nach 9 Stunden zu 85 % frei.

### Beispiel 2:

Die analog nach Beispiel 1 hergestellte Suspension von 3 g Theophyllin und 30 g der dort angegebenen Kunststoffdispersionen, zusammen mit 0,45 Triethylcitat als Weichmacher und 12 g Wasser als Verdünnungsmittel werden, wie dort, noch 5 Min. gerührt, dann jedoch mit einem Rakel mit einer Schlitzöffnung von 500 µm ausgezogen, so daß sich nach 5-stündigem Trocknen bei 50° C ein Film von etwa 200 µm Schichtdicke ergibt. Auf diesen Film wurde in einer Schicht von etwa 50 µ m eine Klebelösung aus Dimethylpolysiloxan in Trichlorfluorethylen (Medical Adhesive Dow Corning 355) aufgetragen. Nach etwa 30 Min. bei Raumtemperatur ist die Kleberschicht aufgetrocknet, und der Film wird nun als Streifen von 2,5 cm Breite und etwa 30 cm Länge noch geschnitten. Nach dem Aufrollen werden mit einem scharfen Messer unter leichtem Druck ovale Scheibchen abgeschnitten, die in der Aufsicht die Form einer Ellypse mit den Durchmessern von 5 und 10 mm aufweisen. Die Scheibchendicke betrug 2,5 mm und enthielt 24,3 mg Wirkstoff pro Dosiseinheit. Im Freigabetest nach USP mit der Paddle-Methode bei 100 Upm und 37° C wird eine Wirkstoffabgabe von 9 % nach 1 Stunde in künstlichem Magensaft gefunden, nach Wechsel auf künstlichen Darmsaft (Phosphatpuffer pH 7,5) und weiteren 5 Stunden Rühren wurden 39 % des Wirkstoffs freigesetzt und nach 24 Stunden 83 %. Scheibchen von 5,0 mm Dicke enthielten 50 mg Wirkstoff und zeigten im gleichen Test nach 1 Stunde in künstlichem Magensaft 5 % und nach weiteren 5 Stunden 23 % bzw. nach 24 Stunden 60 % Wirkstoffabgabe in Phosphatpuffer pH 7,5. Die Filmrolle löste sich zuletzt weitgehend auf.

### Beispiel 3:

600 mg Bisacodyl wurden in 12 g Wasser suspendiert, mit 30 g einer wäßrigen Dispersion von EUDRAGIT NE 30 D (enthalten 9 g Copolymer aus Ethylacrylat und Methylmethacrylat 2:1) vermischt. Daraus wurde durch Trocknen bei 50° C auf einer Fläche von 14 x 20 cm ein Film einer Dicke von 0,2 mm hergestellt. Daraus wurde ein Streifen von 14 cm Länge und 5 cm Breite geschnitten und in Lagen von je 12 mm (= L) wechselseitig gefaltet, bis eine Höhe von 2,5 mm (= H) erhalten wurde (s. das Faltprinzip in Figur 2). Die Filme haften ohne weiteren Kleberzusatz aufeinander. Die letzten 3 cm des Films wurden zum Abschluß um das Laminat herum als Banderole geführt. Aus dem Laminat von insgesamt 5 cm Breite wurden nun Stücke von 1 cm Breite (= B) abgeschnitten, so daß sich die folgenden Maße für die Arzneiform ergaben: L = 12 mm, B = 10 mm und H = 2,5 mm. Die Gewichte der Laminatstückchen lagen zwischen 295 und 325 mg und enthielten je 20 mg Wirkstoff. Beim Freigabetest in künstlichem Magensaft im USP-Paddle-Gerät wurden nach 1 Stunde 18, nach 3 Stunden 29, nach 8 Stunden 40 und nach 24 Stunden über 80 % des Wirkstoffes freigesetzt.

### Beispiel 4:

600 mg Phenylpropanolaminhydrochlorid wurden in 12 g Wasser gelöst und mit 30 g einer wäßrigen Dispersion, enthaltend 9 g Copolymer aus Ethylacrylat und Methylmethacrylat 2:1 vermischt. Ein bei 50° C getrockneter Filmstreifen von 15 cm Länge, 4 cm Breite und 0,2 mm Dicke wurde mit 2 ml Kleberlösung auf Basis von Dimethylpolysiloxan (Dow Medical Adhesive) bestrichen und nochmals längs unterteilt, so daß sich eine Filmbahn von 2 cm Breite und insgesamt 30 cm Länge ergab. Die Filme wurden hintereinander aufgerollt, so daß sich ein Durchmesser der Rolle von 9 mm ergab. Mit einem scharfen Messer wurden nun Scheiben von 4,3 bis 5 mm Dicke abgeschnitten, die ein Gewicht von 302 bis 316 mg aufwiesen und 19,0 bis 20,0 mg Wirkstoff enthielten. Der Wirkstoff wurde beim Freigabetest nach USP - wie in den obigen Beispielen beschrieben - kontinuierlich und innerhalb von 6 Stunden praktisch vollständig freigegeben.

### Beispiel 5:

150 g einer 30 %-igen Dispersion eines Copolymerisats aus 30 Gew.-% Ethylacrylat, 65 Gew.-% Methylmethacrylat und 5 Gew.-% 2-Trimethylammoniumethylmethacrylat-Chlorid (EUDRAGIT® RS 30 D) werden mit einer Vormischung aus 9 g Triethylcitrat als Weichmacher und 3,5 g Polysorbat 80 als Stabilisator in 6,5 g Wasser versetzt und 5 min gerührt. Nun wurden 3 g Polyvinylalkohol als feinkörniges Granulat langsam unter Rühren eingetragen und noch 3 h weiter gerührt. Die viscose Dispersion wurde durch ein Sieb mit 0,1 mm lichter Maschenweite passiert und in einem Filmziehgerät (Fa. Mathes, Typ LTF 142691) auf eine Polyethylen-Folie (®Hostaphan, Typ 931/8478, Fa. Renken GmbH) in einer Schichtdicke von 400 µm auf einer Fläche von ca. 20 x 20 cm aufgetragen, wozu etwa 30 ml Flüssigkeit verbraucht wurden. Nach dem Trocknen 30 min bei 80 °C entsteht ein klarer Film mit einer Schichtdicke von ca. 105 µm, der als Zwischenschicht mit wirkstoffhaltigen Filmen der Beispiele 2 und 4 verwendet werden kann.

## Patentansprüche

1. Laminare Arzneiform mit kontrollierter Wirkstoffabgabe, bestehend aus gerollten oder gefalteten Schichten aus einer Polymerfolie (1), die einen pharmazeutischen Wirkstoff enthält,
**dadurch gekennzeichnet, daß**
die wirkstoffhaltige Polymerfolie (1) aus einem (Meth)acrylat-Copolymer, aus Celluloseacetatphthalat, Hydroxypropylmethylcellulosephthalat, Carboxymethylcellulose und/oder Polyvinylacetatphthalat, gegebenenfalls mit Zusätzen von Weichmachern oder Bindemitteln besteht und
die äußere, den Verdauungssäften zugängliche Oberfläche der wirkstoffhaltigen Polymerfolie im gerollten oder gefalteten Zustand höchstens 25 % ihrer gesamten Oberfläche beträgt und die gerollten oder gefalteten Schichten so aneinander haften, daß die laminare Arzneiform im Freigabetest nach USP 23, Methode A, Apparatus 2 bei 37 °C und 50 Upm in künstlichem Magensaft ihre gerollte oder gefaltete Form über mindestens eine Stunde beibehält und mindestens 30 % des enthaltenen Wirkstoffs im gerollten oder gefalteten Zustand freigesetzt werden.

2. Laminare Arzneiform nach Anspruch 1, **dadurch gekennzeichnet, daß** zusätzlich zur wirkstoffhaltigen Polymerfolie (1) eine Klebeschicht (2) und/oder eine Zwischenschicht (3) enthalten ist.

3. Laminare Arzneiform nach Anspruch 1 oder 2, **dadurch gekennzeichnet, daß** sie von einer Banderole umhüllt ist.

4. Laminare Arzneiform nach Anspruch 2, **dadurch gekennzeichnet, daß** als Klebeschicht Dimethylpolysiloxan, ein Acrylatklebstoff oder ein Klebstoff auf Basis von Naturstoffen verwendet wird.

5. Verfahren zur Herstellung einer laminaren Arzneiform nach einem oder mehreren der vorhergehenden Ansprüche, **dadurch gekennzeichnet, daß** die wirkstoffhaltiger Polymerfolie und ggf. die Zwischen- und/oder Klebeschichten gerollt oder gefaltet werden und die Schnittflächen und Enden jeweils durch heißes Schneiden versintert werden.

## Claims

1. A laminar pharmaceutical form with controlled release of an active substance, comprising rolled or folded layers of a polymer film (1) containing a pharmaceutically active substance,
**characterised in that**
the polymer film (1) containing an active substance consists of a (meth)acrylate copolymer, cellulose acetate phthalate, hydroxypropylmethyl cellulose phthalate, carboxymethylcellulose and/or polyvinyl acetate phthalate, optionally with the addition of plasticisers or binders, and
the outer surface of the polymer film (1) containing the active substance which is exposed to the digestive juices makes up at most 25% of the total surface area thereof in the rolled or folded state and the rolled or folded layers adhere to one another such that the laminar pharmaceutical form retains its rolled or folded shape for at least one hour in a release test according to USP 23, Method A, Apparatus 2 at 37°C and 50 rpm in artificial gastric juice, and at least 30% of the active substance contained therein is released in the rolled or folded state.

2. The laminar pharmaceutical form according to claim 1, **characterised in that** the polymer film (1) containing the active substance further comprises an adhesive layer (2) and/or an intermediate layer (3).

3. The laminar pharmaceutical form according to claim 1 or 2, **characterised in that** it is wrapped by a band.

4. The laminar pharmaceutical form according to claim 2, **characterised in that** the adhesive layer is dimethylpolysiloxane, an acrylate adhesive or an adhesive based on natural substances.

5. Process for producing a laminar pharmaceutical form according to one or more of the preceding claims, **characterised in that** the polymer film containing the active substance and optionally the intermediate and/or adhesive layers are rolled or folded and the cut surfaces and ends are sintered by hot cutting.

## Revendications

1. Forme médicamenteuse stratifiée à libération programmée de substance active, constituée de couches enroulées ou repliées d'une pellicule de polymère (1) qui contient une substance active pharmaceutique,
**caractérisée en ce que**
la pellicule de polymère (1) contenant une substance active est constituée d'un copolymère de (méth)acrylate, d'acétate-phtalate de cellulose, de phtalate d'hydroxypropylméthylcellulose, de carboxyméthylcellulose et/ou de poly(acétate-phtalate de vinyle) éventuellement avec des additions de plastifiants ou de liants et
la surface externe, accessible aux sucs digestifs, de la pellicule de polymère contenant une substance active, représente à l'état enroulé ou replié au maximum 25 % de sa surface totale, et les couches enroulées ou repliées adhèrent les unes aux autres de sorte que la forme médicamenteuse stratifiée, dans l'essai de libération selon USP 23, méthode A, appareil 2 à 37°C et 50 tours/min, conserve pendant au moins une heure sa forme enroulée ou repliée et, à l'état enroulé ou replié, au moins 30 % de la substance active contenue sont libérés.

2. Forme médicamenteuse stratifiée selon la revendication 1,
**caractérisée en ce que**
en plus de la pellicule de polymère (1) contenant une substance active, est(sont) contenue(s) une couche adhésive (2) et/ou une couche intermédiaire (3).

3. Forme médicamenteuse stratifiée selon la revendication 1 ou 2,
**caractérisée en ce qu'**
elle est entourée d'une bande.

4. Forme médicamenteuse stratifiée selon la revendication 1 ou 2,
**caractérisée en ce qu'**
on utilise en tant que couche adhésive du diméthylpolysiloxane, un adhésif acrylate ou un adhésif à base de substances naturelles.

5. Procédé pour la fabrication d'une forme médicamenteuse stratifiée selon une ou plusieurs des revendications précédentes,
**caractérisé en ce que**
la pellicule de polymère contenant une substance active et éventuellement la couche adhésive et/ou la couche intermédiaire sont enroulées ou repliées, et les faces coupées et les extrémités sont frittées chacune par découpage à chaud.
